(19) Europäisches Patentamt

European Patent Office

Office européen des brevets

(11) **EP 0 346 097 B1**

(12) **EUROPEAN PATENT SPECIFICATION**

(45) Date of publication and mention
of the grant of the patent:
**13.05.1998 Bulletin 1998/20**

(51) Int Cl.6: **C08L 33/04**, C08L 5/00,
C11D 3/00, A61K 7/16,
C09D 7/00

(21) Application number: **89305745.5**

(22) Date of filing: **07.06.1989**

(54) **Thickening system**

Verdickungssystem

Système épaississant

(84) Designated Contracting States:
**AT BE CH DE ES FR GB GR IT LI NL SE**

(30) Priority: **08.06.1988 GB 8813552**

(43) Date of publication of application:
**13.12.1989 Bulletin 1989/50**

(73) Proprietors:
• **UNILEVER PLC**
**London EC4P 4BQ (GB)**
Designated Contracting States:
**GB**
• **UNILEVER N.V.**
**3013 AL Rotterdam (NL)**
Designated Contracting States:
**BE CH DE ES FR GR IT LI NL SE AT**

(72) Inventor: **Rennie, George Kerr**
**Bebington Wirral Merseyside L63 9JF (GB)**

(74) Representative: **Elliott, Peter William et al**
**Unilever plc**
**Patent Division**
**Colworth House**
**Sharnbrook**
**Bedford MK44 1LQ (GB)**

(56) References cited:
**EP-A- 0 152 095       GB-A- 1 549 378**
**US-A- 3 658 734       US-A- 4 687 663**

Remarks:
The file contains technical information submitted
after the application was filed and not included in this
specification

## Description

The present invention relates to a synergistic thickening system. More particularly, it relates to a mixture of two different thickening agents which yields a synergistic thickening effect in liquid media.

In very many areas of industry, thickening agents are used to impart a certain rheological behaviour to liquid media. By thickening such liquid media, they can be made more suitable for their end-use, or they can be made more suitable as an intermediate medium in which other substances can be included which need to remain stably suspended or dispersed in the media. By varying the levels of thickening agents, the degree of thickening can be controlled.

A vast number of thickening agents is known in the art, and many of them have found practical application. Since frequently, however, the thickening agent does not contribute anything more than a thickening effect, one attempts to use as little as possible of the thickening agent, since these thickening agents may be rather expensive. In addition, frequently such thickening agents may be adversely affected by other substances present in the liquid media, e.g. electrolyte salts, which imposes restrictions on their use for particular purposes.

Of the known thickening agents, the class of the organic, polymeric thickening agents is perhaps the best known. Among these organic, polymeric thickening agents, the gums feature as a class of widely used thickening agents. Gums or mucilages basically consist of polysaccharides with varying polymerization degrees.

They include the polysaccharide hydrocolloids, which are usually prepared from gums, and they may have been chemically modified, e.g. by partial acetylation, to make them more water-soluble and/or stable in the presence of the other ingredients in the liquid media. Biopolymers also belonging to this class of polysaccharide hydrocolloids are known thickening agents. Typical examples of commercially available, gum-type thickening agents are xanthan gums and their derivatives. These include a partially acetylated xanthan gum, "Kelzan" ex Kelco Company of N.J., USA, Shellflo-XA and Enorflo-XA, xanthan gums ex Shell Chemicals Ltd., and Rhodapol, a xanthan gum ex Rhone-Poulenc SA. A further example is the biopolymer Shellflo S, a succinoglucan ex Shell Chemicals Ltd. Yet other gum-type thickening agents are those derived from guar gums, such as the Jaguar(R) products ex Stein, Hall and Co Inc. and those derived from cellulose such as carboxymethyl or hydroxyethyl cellulose.

The present invention is particularly concerned with the above gum-type thickening agents.

Another group of well-known, organic thickening agents are the synthetic, polymers which include acrylate homo- or coplymers and derivatives thereof. Typical examples of such materials which are suitably cross-linked are the acrylic copolymers sold by National Starch and Chemical Ltd under the trade names EP 1910 and PPE 1042. Other types of such (meth)acrylic homo- and copolymers are certain Carbopol(R)-type, cross-linked carboxyvinyl polymers such as Carbopol(R)-940 ex B.F.Goodrich Co Ltd. Other examples are the Viscalex products ex Allied Colloids, which are emulsions of (meth)acrylic acid copolymers with (meth)acrylate esters, e.g. Viscalex HV 30, Acrysols (ex Rohm & Haas) and Ubatols (ex Stapol).

The present invention is particularly concerned with the synthetic acrylate copolymer-type thickening agents.

Both the gum-type and the acrylic copolymer-type thickening agents have been described in our European Patent Application 0 174 689, published on 19 March 1986, in which representatives of both types have been described for inclusion in shear-thinning liquid cleaning compositions. This publication is hereby incorporated by way of Reference.

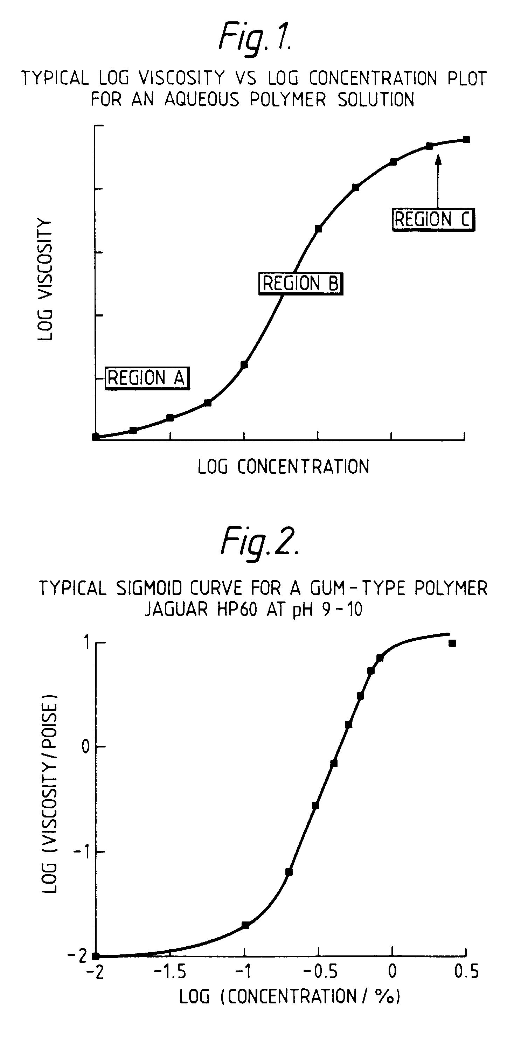

It is well known in the field of organic, polymeric thickening agents that, in general, the viscosity ($\eta$) of a liquid is dependent on the concentration of thickening agent in that liquid. This relationship can be expressed schematically as a sigmoid curve as shown in Figure 1 which is a plot of log $\eta$ vs log c for the thickening agent in the given liquid. While not wishing to be bound by any theory, we believe that in region A the molecules are essentially independent of one another, the viscosity increase arises from disruption of flow of the continuous phase, and the rate of increase is relatively small. In region B the molecules are sufficiently close together to interact, entangle etc., and the viscosity rises very steeply. In region C the units are close packed and increasingly experience compression so that once again the rate of increase in viscosity is relatively small.

Region A is defined as that portion of the sigmoid curve where the viscosity of the total system corresponds approximately to that of the base system and there is a linear relationship between log $\eta$ and log c.

Region B (lower) is defined as that portion of the sigmoid curve which obeys a power law relationship (the coefficient of which is greater than 1) beneath the point of inflexion.

Region B (upper) is defined as that portion of the sigmoid curve which obeys a power law relationship (the coefficient of which is greater than 1) above the point of inflexion.

Region C is defined as that portion of the sigmoid curve where the viscosity of the total system is greater than that of the base system and there is an essentially linear relationship between log $\eta$ and log c.

The point of inflexion for the sigmoid curve is defined as that point where the first derivative of the curve experiences a turning point.

For a given liquid system the position of the sigmoid curve on the log $\eta$ vs log c graph will depend on, for example, polymer type or temperature. It is therefore convenient to describe a given system in terms of a single master curve which represents the actual measured parameter plus a shift factor which superposes the measured curve on the reference curve.

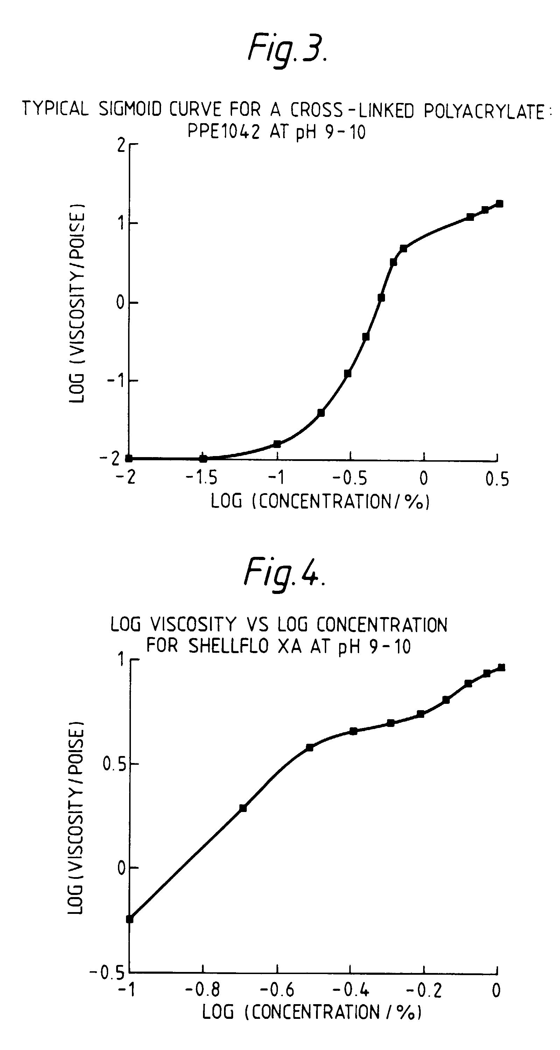

Reference sigmoid curves can be constructed according to the method described in R Simha and L Utracki, J Polymer Sci, A-2, 5, 853 (1967), L Utracki and R Simha, J Polymer Sci, A, 1, 1089 (1963) and R Simha and L Utracki, Rheol. Acta, 12, 455 (1973), for a liquid system comprising a gum-type polymer and for a liquid system comprising an acrylic-type polymer. Typical sigmoid curves for these systems are shown in Figures 2 and 3, which are respectively plots of log $\eta$ vs log c for Jaguar HP60, which is a cellulose derived polymer, and PPE1042 which is a cross-linked acrylic polymer.

US-A-4687663 describes a teeth-cleaning product consisting of two compositions which are mixed together. Some of the compositions include cross-linked polyacrylate identified by the trade mark CARBOPOL, together with hydroxypropylcellulose or hydroxyethylcellulose.

US-A-3658734 discloses the use of guar gum and polyacrylamide in combination as thickening agents in fluids for use in oil well drilling. The polyacrylamide is depicted as a linear polymer. The document teaches that the guar gum and polyacrylamide exhibit synergistic thickening.

GB-A-1549378 describes oil-well drilling fluids containing polyacrylate (often a linear polymer) and polysaccharide.

According to the present invention there is provided a composition comprising a liquid system which contains an aqueous liquid medium including 0.05 to 20 wt% of detergent, and a thickening mixture dispersed in the liquid medium, which thickening mixture is present in an amount from 0.01 to 5% by weight with respect to the liquid system and comprises a gum-type polymer which is a polysaccharide and an acrylic-type polymer which is selected from the group consisting of synthetic cross-linked acrylate and methacrylate homopolymers and copolymers and mixtures and derivatives thereof,

wherein the gum-type polymer and the acrylic-type polymer are present in a weight ratio of gum-type polymer to acrylic-type polymer of between 50:1 and 1:100,

and wherein each said polymer, when in the liquid medium in the absence of the other said polymer, has a relationship between concentration and viscosity, measured at a shear rate of 10 sec$^{-1}$ and a temperature of 25°C such that a graph of log (viscosity) against log (concentration) is a sigmoid curve having a portion where, over a range of concentration the gradient of the curve of log (viscosity) against log (concentration) is constant or increases with log (concentration), and the amount of each of the said polymers is such that the concentration thereof in the liquid medium lies within the said range over which the gradient of the polymer's said curve of log (viscosity) against log (concentration) is constant or increasing,

the liquid system having a viscosity of at least 20 cPs at a shear rate of 10 sec$^{-1}$ greater than that of the liquid medium in the absence of the said thickening mixture, said liquid system containing either no disperse phase, or a disperse phase which is particulate solid material.

It has been found that in liquid compositions so formulated a synergistic thickening effect can be achieved. We have found that many embodiments of the liquid systems according to the invention show synergistic thickening together with shear-thinning, thus providing good flow properties. It is to be understood however that the liquid system of the present invention also extends to pastes thickened by the present thickening mixture.

The synergistic thickening mixture of the present invention can contain more than one of each of the two types of thickening agent.

In the ideal case the sigmoid curves for each polymer will be as shown in Figure 1. The lower portion of the curve from which the present polymers must be selected thus corresponds in the ideal case to region A and region B (lower), subject to the limitation that sufficient polymers must be present to effect an increase in the system of at least 20 cPs at a shear rate of 10 sec $^{-1}$. In practice when the present curves are constructed for a variety of polymers not all polymers follow an ideal sigmoid curve. An overall sigmoid shape can be discerned allowing the presently defined lower portion to be identified i.e. that portion having a constant or increasing gradient and extending between a point near the origin along the curve until the gradient begins to decrease. In some instances however region C may in effect be non-existent as the viscosity at such concentrations may be too high to measure readily or alternatively region C may include a second point of inflexion. In the latter instance it is only the lower portion of the curve up until the first point of inflexion from which the polymer should be selected. Another non-ideal variant in the shape of the sigmoid curve may include a region of substantially constant gradient between region B (lower) and region B (upper). Such region would fall within the present definition of the lower portion of the curve from which the present polymers must be selected.

It should be noted moreover that the sigmoid curves to be employed are those measured according to the liquid medium in question, ie the log vicosity vs. log concentration plot of each polymer is that of the polymer in the presence of any other ingredients which might be present in the liquid medium.

The relative weight ratio between the gum-type and the acrylic copolymer-type thickening agent in the thickening mixture ranges from 50:1 to 1:100, preferably from 20:1 to 1:25 and more preferably from 5:1 to 1:10. The amount of thickening mixture used for thickening liquid media ranges between 0.01 and 5 wt%, more preferably from 0.1 to 5% by weight of the liquid system , usually from 0.25 to 5% by weight, and preferably from 0.5 to 1.5% by weight of the liquid system.

Examples of mixtures of thickening agents embodying the present invention are mixtures of Shellflo-XA with PPE 1042, Shellflo-XA with Carbopol 940, and Shellflo-XA with Viscalex HV30 in appropriate liquid systems.

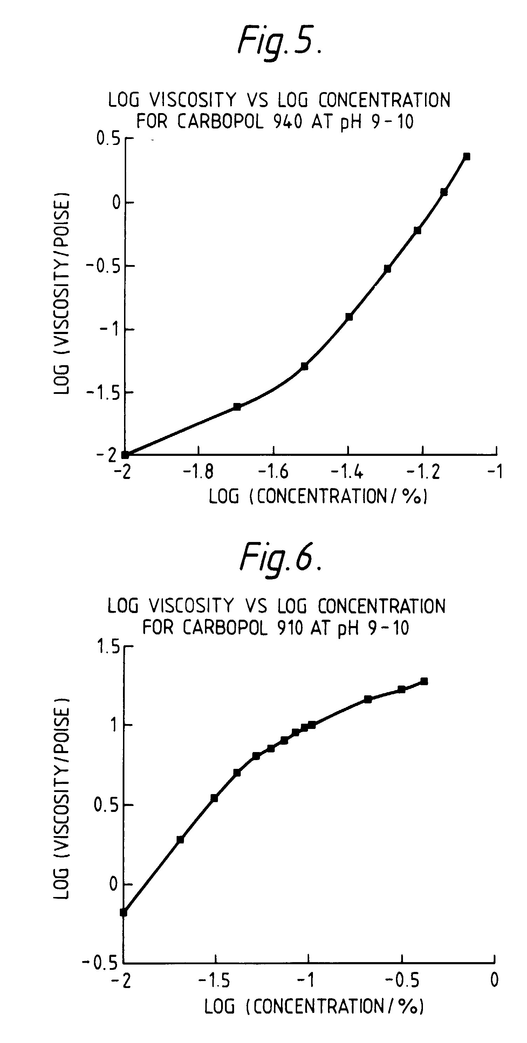

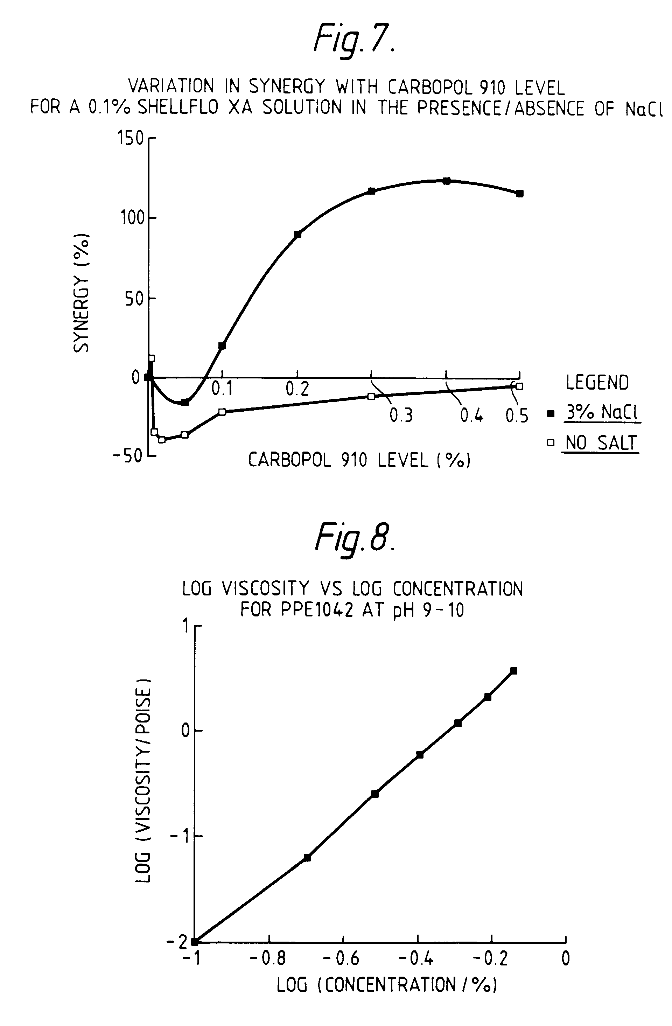

The invention has the added advantage that by altering the variables of a given system it is possible to manipulate otherwise unsuitable polymers into the A region or B (lower) region of their sigmoid curve and obtain the synergistic benefit. For example a mixture of 0.1% Shellflo XA (Figure 4) with 0.04% Carbopol 940 (Figure 5) in aqueous dispersion at pH 9.5 gives a synergistic increment of approximately 140% at 25°C, Figures 4 and 5 being respectively log $\eta$ vs log c plots in aqueous media at pH 9 to 10 for Shellflo XA and Carbopol 940. Alternatively a mixture of 0.1% Shellflo XA with 0.2% Carbopol 910 in aqueous dispersion at pH 9.5 gives a synergistic increment of approximately -30%. The Carbopol 940 therefore gives the synergistic benefit when mixed with Shellflo XA while Carbopol 910 does not for this particular system. It is believed that the explanation for this difference in behaviour lies in the observation that at 0.2% concentration Carbopol 910 is in the C region of its sigmoid curve for this system. Figure 6 is a plot of log $\eta$ vs log c for Carbopol 910 in aqueous medium at pH 9 to 10. It follows that if A or B (lower) region behaviour could be induced in the Carbopol 910 by changing the system then the synergistic benefit would be obtained. The addition of 3% salt to a 0.1% Shellflo XA/0.2% Carbopol 910 mixture achieves this and a synergistic increment of 95% is seen. These results are given in Figure 7 which are plots of synergy in % vs concentration of Carbopol 910 in % for a 0.1% Shellflo XA solution with and without 3% NaCl.

Thus the present liquid systems can include a liquid medium which includes an electrolyte. A preferred level of electrolyte is 0.1 to 10 wt% electrolyte with respect to the liquid medium.

For a particular system the synergistic increment can be calculated according to the equation given below

$$S = \left[ \frac{\eta(P_1 + P_2)}{\eta P_1 + \eta P_2} - 1 \right] \times 100$$

where

S = the synergistic increment
$\eta(P_1 + P_2)$ = the viscosity of a mixture of polymers $P_1$ and $P_2$
$\eta P_1$ = the viscosity of polymer $P_1$
$\eta P_2$ = the viscosity of polymer $P_2$

Preferably the thickening mixture imparts a synergistic increment S of at least 5%, more preferably a synergistic increment of at least 10%, even more preferably a synergistic increment of at least 50%.

The synergistic mixture of the thickening agents according to the present invention can be used for a variety of liquid systems to be thickened. If desired the liquid medium can contain a dispersed solid phase which could be for example a suspended particulate ingredient.

Examples of the present liquid systems include liquid detergent and cleaning compositions. Suitably such liquid systems contain 0.05 to 20 wt%, more suitably 0.1 to 15 wt%, even more suitably 2 to 10 wt% of a detergent active material selected from the group comprising anionic, nonionic, zwitterionic, cationic detergents and appropriate mixtures thereof. Particularly suitable detergent active materials include soap and synthetic materials such as alkylbenzenesulphonates, alkanesulphonates, alkylsulphates, alkylethersulphates and mixtures thereof, all of which would be compatible for use with an anionic acrylic type polymer. If desired the liquid detergent and cleaning compositions can contain stably suspended particulate detergent ingredients and/or particulate abrasive materials. Other ingredients commonly encountered in such compositions may also be included, such as builders including polymeric builders, sequestering agents, dyes, preservatives, perfumes, bleaches, bleach activators, solvents, enzymes, foam controlling agents and hydrotropes. The liquid medium of the composition is usually an aqueous medium.

Particularly in the field of aqueous, liquid abrasive cleaning compositions, which generally contain from 1% up to 70% of particulate abrasive material, the mixture according to the present invention is of surprising benefit. Such compositions, when containing the present synergistic thickening mixture, can show excellent physical stability as well as improved cleaning and reduced damage, particularly on soft substrates, and can show an improved rinsability when compared with current liquid abrasive cleaners.

It has moreover been found that liquid abrasive cleaners embodying the present invention and containing 30 to 40 wt%, preferably 35 wt%, abrasive particulate material can have the same viscosity as conventionally formulated liquid abrasive cleaners containing about 50 wt% particulate abrasive material. Use of the present thickening system in liquid abrasive cleaners can thus permit more flexibility in formulation. A preferred thickening system for use in liquid abrasive cleaners comprises, with respect to the final liquid system, 0.05 to 1.0 wt% synthetic cross-linked acrylate polymer and 0.05 to 0.7 wt% xanthan gum derivative or mixtures thereof. Suitably a liquid abrasive cleaner contains 0.1 to 10 wt% nonionic surfactant. It is to be understood that the present invention extends to a liquid abrasive cleaner containing 1 to 70 wt% particulate abrasive material and 0.5 to 1.5 wt% thickening mixture, the thickening mixture preferably comprising with respect to the final composition 0.05 to 1.0 wt% synthetic cross-linked acrylate polymer and 0.05 to 0.5 wt% xanthan gum and/or xanthan gum derivative, the cleaner optionally containing 0.1 to 10 wt% nonionic detergent.

Similarly, the synergistic mixture can be used in liquid fabric washing compositions to impart improved suspending properties thereto. A specific area for application is in the field of lavatory cleaners. It has been found that such mixtures according to the invention may have improved shear-thinning and drainage behaviour when compared with mixtures containing acrylate polymers alone. It is to be understood that the present invention extends to a cleaning composition containing 0.02 preferably 0.05 or 0.01 up to 15 or 20 wt% detergent selected form cationic, anionic, nonionic, zwitterionic surfactants and mixtures thereof and 0.5 to 1.5 wt% thickening mixture, the thickening mixture preferably comprising 0.05 to 0.5 wt% synthetic cross-linked acrylate polymers and 0.05 to 1.0 wt% xanthan gum and/or xanthan gum derivative.

Another area of use of the synergistic mixture is the area of personal products such as shampoos, shower and bath gels, lotions, creams, and products for dental care. In this respect, it has surprisingly been found that the inclusion of the synergistic mixture in toothpastes can provide toothpastes which have a higher gloss, a cleaner ribbon break and a smoother texture than toothpastes which have been thickened with the aid of current thickeners, e.g. sodium carboxymethyl cellulose. The present invention thus beneficially extends to a liquid system in the form of toothpaste containing 0.25 to 5 wt% of thickening mixture comprising preferably, with respect to the final composition, 0.2 to 3 wt% synthetic cross-linked acrylate polymer 0.05 to 2 wt% xanthan gum and/or xanthan gum derivative.

In cleaners, the synergistic mixture can promote cling to vertical surfaces for longer contact time and can make possible formulation of gel-type acid and alkali cleaners for industrial applications.

The invention can be used in gel-type "cling" applications. For example, acid/neutral cleaners, de-rusting and iron-stain removal in baths, denture fixatives.

The synergistic mixture can be added to the liquid media to be thickened in several ways, but care should be taken to avoid excessive aeration. We have found that a preferred way of incorporating the synergistic mixture in e.g. a liquid abrasive cleaning composition comprises the steps of adding the particulate abrasive material to a dispersion of the gum-type thickening agent at alkaline pH, and subsequently adding the acrylic copolymer-type thickening agent in pre-neutralized form to the resulting dispersion and thereafter adding the remaining ingredients of the composition.

The invention will further be illustrated by way of example.

EXAMPLE I

The viscosity of various thickening mixtures and individual components of these mixture was measured under the following conditions:

| | |
|---|---|
| Shear rate | $10 \text{ sec}^{-1}$ and $110 \text{ sec}^{-1}$ |
| Temperature | 25°C |
| Water | distilled water |
| Viscosity | viscosity in mPaS as measured on a Haake RV2 viscometer |

The thickening agents used were Shellflo-XA (Figure 4) and PPE 1042 (Figure 8) at a pH of 10.
The following results were obtained:

| | | Viscosity in mPaS | |
|---|---|---|---|
| % Shellflo-XA | % PPE 1042 | at $10 \text{ sec}^{-1}$ | at $110 \text{ sec}^{-1}$ |
| 0 | 0 | 1 | 1 |
| 0.01 | 0 | 9.8 | 4.4 |
| 0.1 | 0 | 86.2 | 20 |
| 0.2 | 0 | 206 | 38.4 |

(continued)

| % Shellflo-XA | % PPE 1042 | Viscosity in mPaS | |
| | | at 10 sec$^{-1}$ | at 110 sec$^{-1}$ |
|---|---|---|---|
| 0 | 0.5 | 24 | 15.4 |
| 0 | 0.7 | 74.9 | 36 |
| 0 | 0.9 | 180 | 84.7 |
| 0.01 | 0.5 | 52 | 26 |
| 0.1 | 0.5 | 250 | 68.7 |
| 0.2 | 0.5 | 420 | 100.4 |
| 0.01 | 0.7 | 115 | 46.7 |
| 0.1 | 0.7 | 298 | 92.3 |
| 0.2 | 0.7 | 610 | 155.6 |
| 0.01 | 0.9 | 200 | 76.9 |
| 0.1 | 0.9 | 500 | 146.2 |
| 0.2 | 0.9 | 800 | 207.4 |

EXAMPLE 2

Mixtures of 0.1% Shellflo-XA with varying amounts of Carbopol 940 at neutral pH were tested according to Example 1 at a shear rate of 10 sec$^{-1}$. The following results were obtained:

| % Carbopol 940 | % increase in viscosity |
|---|---|
| 0 | 0 |
| 0.01 | 20 |
| 0.02 | 65 |
| 0.03 | 110 |
| 0.04 | 140 |
| 0.05 | 140 |
| 0.06 | 80 |
| 0.07 | 35 |
| 0.08 | 10 |

EXAMPLE 3

Repeating Example 2, but using varying amounts of Viscalex HV 30 at neutral pH instead of Carbopol 940, gave the following results:

| % Viscalex HV 30 | % increase in viscosity |
|---|---|
| 0 | 0 |
| 0.1 | 15 |
| 0.2 | 65 |
| 0.3 | 75 |
| 0.4 | 35 |
| 0.5 | 5 |

EXAMPLE 4

Xanthan gum and cross linked polyacrylate were used to thicker sea water having the following ionic composition:

6

| | |
|---|---|
| $Na^+$ | 10900 mg/l |
| $Ca^{2+}$ | 428 mg/l |
| $Mg^{2+}$ | 1368 mg/l |
| $K^+$ | 460 mg/l |
| $Sn^{2+}$ | 8 mg/l |
| $Cl^-$ | 18700 mg/l |
| $SO_4^{2-}$ | 2960 mg/l |
| $HCO_3^-$ | 124 mg/l |

Experiments performed using a mixture of a xanthan gum (Kelzan XC ex Kelco Company) and a cross-linked polyacrylate (PPE 1087 ex National Starch) to thicken the above sea water composition gave a synergistic increment of 600% when the sea water contained 0.3 wt% xanthan gum (dry) and 1.25 wt% cross-linked polyacrylic polymer. Table 1 lists the synergy S, as defined above, in percentage terms vs concentration of polyacrylic polymer in wt% for a sea water composition containing a constant amount of xanthan gum at 0.3 wt% and clearly shows the synergy in thickening for compositions containing between 0.75 and 1.5 wt%, peaking at 1.25 wt%, cross-linked polyacrylic polymer.

| SYNERGY BETWEEN PPE 1087 AND XANTHAN GUM IN SEA WATER THICKENING | |
|---|---|
| % PPE 1087 (DRY) | % SYNERGY |
| 0.75 | 140 |
| 1.00 | 300 |
| 1.25 | 600 |
| 1.5 | 110 |

## EXAMPLE 5

The following liquid abrasive cleaners were prepared:

| | % by weight | | |
|---|---|---|---|
| | A | B | C |
| PPE 1042 | 0.1 | 0.5 | 0.9 |
| Shellflo-XA | 0.2 | 0.2 | 0.2 |
| Topped $C_9$-$C_{11}$ linear alcohol condensed with 5 moles of ethylene oxide | 1.5 | 1.5 | 1.5 |
| Perfume | 0.7 | 0.7 | 0.7 |
| Particulate calcite | 50 | 50 | 50 |
| Water | q.s. | q.s. | q.s. |

Glossy, black, ceramic tiles were cleaned with these products and, after rinsing and drying, they were visually assessed as to formation of streaks thereon. As control, a commercially available, liquid abrasive cleaner was used. The following results were obtained:

| | Applied in water of 40°FH at 45°C | Applied in demineralized water | Used neat |
|---|---|---|---|
| A | No streaks | No streaks | No streaks |
| B | " | " | " |
| C | " | " | " |
| Control | Streaky | Slightly streaky | White streaky film |

On storing these products A, B and C for 6 months at ambient temperature, no phase separation was observed. In cleaning tests, these products were twice as effective as the control in removing hydrophobic soil from Perspex substrates, and reduced damage to the Perspex surface by half.

## EXAMPLE 6

The following liquid abrasive cleaning composition was prepared by mixing the ingredients in the given order of addition:

| Order of ingredient addition | | % Composition |
|---|---|---|
| 1. | Demineralized water | to 100 |
| 2. | Shellflo-XA (at pH 9.8) | 0.02 |
| 3. | Particulate calcite | 35.0 |
| 4. | Titanium dioxide | 0.5 |
| 5. | PPE 1042 (at pH 9.8) | 0.5 |
| 6. | Topped $C_9$-$C_{11}$ linear alcohol condensed with 5 moles of ethylene oxide | 1.5 |
| 7. | Preservative | 0.05 |
| 8. | Perfume | 0.2 |

The viscosity of this product, when made, was 1140 mPAS, and after two weeks' storage at room temperature and at 37°C, the viscosity was 1086 and 942 mPAS, respectively. The pH of the product, when made, was 9.84.

## EXAMPLE 7

The following toothpastes were prepared:

| | | % by weight | |
|---|---|---|---|
| Silica (Gasil 200) | | 12 | - |
| Alumina (AF 240) | | - | 50 |
| Sorbitol | | 45 | 27 |
| Sodium lauryl sulphate | | 1.7 | 1.7 |
| Sodium dodecyl benzene sulphonate | | 0.5 | 0.5 |
| Sodium monofluorophosphate | | 0.76 | 0.76 |
| Saccharin | | 0.2 | 0.2 |
| Tiona G | | 1 | 1 |
| Flavour | | 1 | 1 |
| PPE 1042 | | 1.8 | 0.7 |
| Shellflo-XA | | 0.2 | 0.1 |
| Viscosity in PaS | at shear rate | | |
| (at room temperature, after 5 weeks) | 0.1 sec | 400 | 900 |
| | 10 sec | 60 | 70 |

These toothpastes were glossier and smoother than a commercially available toothpaste thickened with a Carbopol/sodium carboxymethyl cellulose mixture.

## EXAMPLE 8

The following lavatory cleaners were prepared:

| | A | B | C | D | E |
|---|---|---|---|---|---|
| PPE 1042 | | | | | 4.55 |
| Shellflo-XA | 0.2 | 0.2 | 0.25 | 0.25 | - |
| Carbopol 940 | 0.11 | 0.14 | 0.14 | 0.17 | - |
| anionic active[1] | 0.25 | | | | |

1 is alkyl benzene sulphonate

(continued)

|  | A | B | C | D | E |
|---|---|---|---|---|---|
| anionic active[2] | 0.25 | | | | |
| nonionic active[3] | 1.5 | | All levels | | |
| Formalin | 0.75 | | unchanged | | |
| Yellow dye | 0.006 | | | | |
| Blue dye | 0.005 | | | | |
| Perfume | 0.7 | | | | |
| Borax | 0.1 | | | | |
| Water | to 100% | | | | |
| Synergy % | 182 | 259 | 252 | 206 | - |
| Residual Mass % after 40 mins. | 20 | 28 | 32 | 35 | 28 |

2 is lauryl ether sulphonate

3 is alkylethoxylate of $C_9$-$C_{11}$ chain length substituted with 8EO per molecule.

Claims

1. A composition comprising a liquid system which contains an aqueous liquid medium including 0.05 to 20 wt% of detergent, and a thickening mixture dispersed in the liquid medium, which thickening mixture is present in an amount from 0.01 to 5% by weight with respect to the liquid system and comprises a gum-type polymer which is a polysaccharide and an acrylic-type polymer which is selected from the group consisting of synthetic cross-linked acrylate and methacrylate homopolymers and copolymers and mixtures and derivatives thereof,

   wherein the gum-type polymer and the acrylic-type polymer are present in a weight ratio of gum-type polymer to acrylic-type polymer of between 50:1 and 1:100,
   and wherein each said polymer, when in the liquid medium in the absence of the other said polymer, has a relationship between concentration and viscosity, measured at a shear rate of 10 sec$^{-1}$ and a temperature of 25°C such that a graph of log (viscosity) against log (concentration) is a sigmoid curve having a portion where, over a range of concentration the gradient of the curve of log (viscosity) against log (concentration) is constant or increases with log (concentration), and the amount of each of the said polymers is such that the concentration thereof in the liquid medium lies within the said range over which the gradient of the polymer's said curve of log (viscosity) against log (concentration) is constant or increasing,
   the liquid system having a viscosity of at least 20 cPs at a shear rate of 10 sec$^{-1}$ greater than that of the liquid medium in the absence of the said thickening mixture, said liquid system containing either no disperse phase, or a disperse phase which is particulate solid material.

2. A composition according to claim 2 where the weight ratio of gum-type polymer to acrylic-type polymer is between 20:1 and 1:25.

3. A composition according to either one of the preceding claims wherein the thickening mixture is present at a level between 0.01 and 1 wt% with respect to the liquid system.

4. A composition according to claim 3 wherein the thickening mixture is present at a level between 0.25 and 5 wt% with respect to the liquid system.

5. A composition according to any one of the preceding claims wherein the gum-type polymer is selected from the group comprising polysaccharides and polysaccharide hydrocolloids and derivatives thereof, xanthan gums and derivatives thereof, guar gums and derivatives thereof, succinoglucan gums and derivatives thereof and cellulose derivatives, and mixtures thereof.

6. A composition according to any one of the preceding claims wherein the liquid medium includes an electrolyte.

7. A composition according to claim 6 wherein the liquid medium contains 0.1 to 10 wt% electrolyte.

8. A composition according to any one of the preceding claims wherein the thickening mixture imparts a synergistic increment S of at least 5% wherein S is defined as

$$S = \left\{ \frac{\eta(P_1+P_2) - 1}{\eta P_1 + \eta P_2} \right\} \times 100$$

where

$\eta(P_1+P_2) =$     viscosity of a mixture of polymers $P_1$ and $P_2$ as measured for the said liquid medium

$\eta P_1 =$     viscosity of polymer $P_1$ as measured for the said liquid medium

$\eta P_2 =$     viscosity of polymer $P_2$ as measured for the said liquid medium

9. A composition according to any one of the preceding claims wherein, with respect to the liquid system, the liquid medium includes 0.05 to 15 wt% detergent and the thickening mixture is present at a level of 0.5 to 1.5 wt% and comprises a mixture of a synthetic cross-linked acrylate polymer and a xanthan gum and/or xanthan gum derivative.

10. A composition according to any one of the preceding claims wherein the liquid medium contains a dispersed particulate solid in an amount which is from 1 to 70% by weight of the composition.

11. A composition according to claim 10 wherein the liquid medium contains 1 to 70 wt% particulate abrasive material.

12. A composition according to claim 11 wherein with respect to the liquid system the thickening mixture comprises 0.05 to 1.0 wt% synthetic cross-linked acrylate polymer and 0.05 to 0.5 wt% xanthan gum and/or xanthan gum derivative.

13. A composition according to any one of the preceding claims in the form of a toothpaste, the thickening mixture with respect to the liquid system comprising 0.5 to 3 wt% synthetic cross-linked acrylate polymer and 0.05 to 0.5 wt% xanthan gum and/or xanthan gum derivative.

14. A composition according to any one of claims 1 to 8 wherein the liquid system is in a form selected from the group comprising shampoos, shower and bath gels.

**Patentansprüche**

1. Mittel, umfassend ein flüssiges System, das ein wässeriges flüssiges Medium, einschließlich 0,05 bis 20 Gew.-% eines Waschmittels und ein verdickendes, in dem flüssigen Medium dispergiertes Gemisch enthält, wobei das verdickende Gemisch in einer Menge von 0,01 bis 5 Gew.-% hinsichtlich des flüssigen Systems vorliegt und ein Polymer vom Gummityp, nämlich ein Polysaccharid und ein Polymer vom Acryltyp, das aus der Gruppe, bestehend aus synthetischen, vernetzten Acrylat- und Methacrylat-Homopolymeren und -Copolymeren und Gemischen und Derivaten davon, ausgewählt ist, umfaßt,

wobei das Polymer vom Gummityp und das Polymer vom Acryltyp in einem Gewichtsverhältnis von Polymer vom Gummityp zu Polymer vom Acryltyp zwischen 50:1 und 1:100 vorliegen, und wobei das Polymer in dem flüssigen Medium in Abwesenheit des anderen genannten Polymers eine Beziehung zwischen Konzentration und Viskosität aufweist, gemessen bei einer Schergeschwindigkeit von 10 s$^{-1}$ und bei einer Temperatur von 25°C, so daß eine Kurve von Log (Viskosität) gegen Log (Konzentration) eine sigmoide Kurve ist mit einem Teil, bei dem über einen Konzentrationsbereich die Steigung der Kurve von Log (Viskosität) gegen Log (Konzentration) konstant ist oder mit Log (Konzentration) steigt und die Menge von jedem der Polymere derart ausgelegt ist, daß die Konzentration davon in dem flüssigen Medium innerhalb des genannten Bereiches, über den die Steigung der genannten Kurve des Polymers von Log (Viskosität) gegen Log (Konzentration) konstant ist oder ansteigt, liegt,

wobei das flüssige System bei einer Schergeschwindigkeit von 10 s$^{-1}$ eine Viskosität von mindestens 20 cPs größer als jene des flüssigen Mediums in Abwesenheit des verdickenden Gemisches aufweist und das flüssige System entweder keine disperse Phase oder eine disperse Phase, die ein teilchenförmiges festes Material ist, enthält.

2. Mittel nach Anspruch 2, wobei das Gewichtsverhältnis von Polymer vom Gummityp zu Polymer vom Acryltyp zwischen 20:1 und 1:25 liegt.

3. Mittel nach einem der vorangehenden Ansprüche, wobei das verdickende Gemisch in einer Menge zwischen 0,01 und 1 Gew.-% hinsichtlich des flüssigen Systems vorliegt.

4. Mittel nach Anspruch 3, wobei das verdickende Gemisch in einer Menge zwischen 0,25 und 5 Gew.-% hinsichtlich des flüssigen Systems vorliegt.

5. Mittel nach einem der vorangehenden Ansprüche, wobei das Polymer vom Gummityp ausgewählt ist aus der Gruppe, umfassend aus Polysacchariden und Polysaccharidhydrokolloiden und Derivaten davon, Xanthangummen und Derivaten davon, Guargummen und Derivaten davon, Succinoglucangummen und Derivaten davon und Cellulosederivaten und Gemischen davon.

6. Mittel nach einem der vorangehenden Ansprüche, wobei das flüssige Medium einen Elektrolyt einschließt.

7. Mittel nach Anspruch 6, wobei das flüssige Medium 0,1 bis 10 Gew.-% Elektrolyt enthält.

8. Mittel nach einem der vorangehenden Ansprüche, wobei das verdickende Gemisch einen synergistischen Zuwachs S von mindestens 5% verleiht, wobei S definiert ist als

$$ S = \left[ \frac{\eta\,(P_1 + P_2)}{\eta\,P_1 + \eta\,P_2} - 1 \right] \times 100 $$

worin

$\eta(P_1+P_2)$ = viskosität eines Gemisches von Polymeren $P_1$ und $P_2$, gemessen für das flüssige Medium,
$\eta P_1$ = Viskosität von Polymer $P_1$, gemessen für das flüssige Medium
$\eta P_2$ = Viskosität von Polymer $P_2$, gemessen für das flüssige Medium.

9. Mittel nach einem der vorangehenden Ansprüche, wobei hinsichtlich des flüssigen Systems das flüssige Medium 0,05 bis 15 Gew.-% Waschmittel einschließt und das verdikkende Gemisch in einer Menge von 0,5 bis 1,5 Gew.-% vorliegt und ein Gemisch aus synthetischem, vernetztem Acrylatpolymer und einem Xanthangummi und/oder Xanthangummiderivat umfaßt.

10. Mittel nach einem der vorangehenden Ansprüche, wobei das flüssige Medium einen dispergierten, teilchenförmigen Feststoff in einer Menge von 1 bis 70 Gew.-% des Mittels enthält.

11. Mittel nach Anspruch 10, wobei das flüssige Medium 1 bis 70 Gew.-% teilchenförmiges Scheuermaterial enthält.

12. Mittel nach Anspruch 11, wobei hinsichtlich des flüssigen Systems das verdickende Gemisch 0,05 bis 1,0 Gew.-% synthetisches vernetztes Acrylatpolymer und 0,05 bis 0,5 Gew.-% Xanthangummi und/oder Xanthangummiderivat umfaßt.

13. Mittel nach einem der vorangehenden Ansprüche in Form einer Zahnpaste, wobei das verdickende Gemisch hinsichtlich des flüssigen Systems 0,5 bis 3 Gew.-% synthetisches vernetztes Acrylatpolymer und 0,05 bis 0,5 Gew.-% Xanthangummi und/oder Xanthangummiderivat umfaßt.

**14.** Mittel nach einem der Ansprüche 1 bis 8, wobei das flüssige System in einer Form, ausgewählt aus der Gruppe, bestehend aus Shampoos, Dusch- und Badegels, vorliegt.

**Revendications**

**1.** Une composition comprenant un système liquide, qui contient un milieu liquide aqueux comprenant 0,05 à 20 % en masse de détergent, et un mélange épaississant dispersé dans le milieu liquide, lequel mélange épaississant est présent selon une quantité comprise entre 0,01 et 5 % en masse, par rapport au système liquide, et comprend un polymère de type gomme, qui est un polysaccharide, et un polymère de type acrylique, qui est sélectionné parmi le groupe composé d'homopolymères et de copolymères d'acrylate synthétique réticulé et de méthacrylate et de mélanges et de dérivés de ceux-ci, dans lequel le polymère de type gomme et le polymère de type acrylique sont présents selon un rapport de masse entre polymère de type gomme et polymère de type acrylique compris entre 50:1 et 1:100, et dans lequel chacun desdits polymères, lorsqu'il est dans le milieu liquide en l'absence de l'autre desdits polymères, présente un rapport entre concentration et viscosité, mesuré à un taux de cisaillement de 10 sec$^{-1}$ et à une température de 25°C, tel qu'un graphique du logarithme (viscosité) en fonction du logarithme (concentration) est une courbe sigmoïde présentant une portion, dans laquelle, au-delà d'une gamme de concentration, le gradient de la courbe du logarithme (viscosité) en fonction du logarithme (concentration) est constant ou augmente avec le logarithme (concentration), et la quantité de chacun desdits polymères est telle, que la concentration de celui-ci dans le milieu liquide est comprise dans ladite gamme au-delà de laquelle le gradient de la courbe dudit polymère du logarithme (viscosité) en fonction du logarithme (concentration) est constant ou croissant, le système liquide présentant une viscosité d'au moins 20 CPs à un taux de cisaillement de 10 sec$^{-1}$ supérieur à celui du milieu liquide en l'absence dudit mélange épaississant, ledit système liquide contenant, soit aucune phase dispersée, soit une phase dispersée qui est une matière solide particulaire.

**2.** Une composition selon la revendication 1, dans laquelle le rapport de masse entre le polymère de type gomme et le polymère de type acrylique est compris entre 20:1 et 1:25.

**3.** Une composition selon l'une quelconque des revendications précédentes, dans laquelle le mélange épaississant est présent selon un niveau compris entre 0,01 et 1 % en masse par rapport au système liquide.

**4.** Une composition selon la revendication 3, dans laquelle le mélange épaississant est présent selon un niveau compris entre 0,25 et 5 % en masse par rapport au système liquide.

**5.** Une composition selon l'une quelconque des revendications précédentes, dans laquelle le polymère de type gomme est sélectionné parmi le groupe composé de polysaccharides et d'hydrocolloïdes de polysaccharides et de dérivés de ceux-ci, de gommes de xanthane et de dérivés de celles-ci, de gommes de guar et de dérivés de celles-ci, de gommes de succinoglucane et de dérivés de celles-ci et de dérivés de cellulose, et de mélanges de ceux-ci.

**6.** Une composition selon l'une quelconque des revendications précédentes, dans laquelle le milieu liquide comprend un électrolyte.

**7.** Une composition selon la revendication 6, dans laquelle le milieu liquide contient 0,1 à 10 % en masse d'électrolyte.

**8.** Une composition selon l'une quelconque des revendications précédentes, dans laquelle le mélange épaississant confère une augmentation de la synergie S d'au moins 5 %, où S est définie de la façon suivante :

$$S = \left[ \frac{\eta\,(P_1 + P_2)}{\eta P_1 + \eta P_2} \right] - 1 \quad \times 100$$

où

$\eta\,(P_1 + P_2)$ correspond à la viscosité d'un mélange composé des polymères $P_1$ et $P_2$, tel que mesuré pour ledit milieu liquide,
$\eta P_1$ correspond à la viscosité du polymère $P_1$, tel que mesuré pour ledit milieu liquide,

η P$_2$ correspond à la viscosité du polymère P$_2$, tel que mesuré pour ledit milieu liquide.

9. Une composition selon l'une quelconque des revendications précédentes, dans laquelle, par rapport au système liquide, le milieu liquide contient 0,05 à 15 % en masse d'un détergent et le mélange épaississant est présent selon un niveau compris entre 0,5 et 1,5 % en masse et comprend un mélange composé d'un polymère d'acrylate réticulé synthétique et d'une gomme de xanthane et/ou d'un dérivé de gomme de xanthane.

10. Une composition selon l'une quelconque des revendications précédentes, dans laquelle le milieu liquide contient un solide particulaire dispersé selon une quantité comprise entre 1 et 70 % en masse de la composition.

11. Une composition selon la revendication 10, dans laquelle le milieu liquide contient 1 à 70 % en masse d'une matière abrasive particulaire.

12. Une composition selon la revendication 11, dans laquelle, par rapport au système liquide, le mélange épaississant comprend 0,05 à 1,0 % en masse d'un polymère d'acrylate réticulé synthétique et 0,05 à 0,5 % en masse d'une gomme de xanthane et/ou d'un dérivé de gomme de xanthane.

13. Une composition selon l'une quelconque des revendications précédentes, qui se présente sous forme d'un dentifrice, le mélange épaississant comprenant, par rapport au système liquide, 0,5 à 3 % en masse d'un polymère d'acrylate réticulé synthétique et 0,05 à 0,5 % en masse d'une gomme de xanthane et/ou d'un dérivé de gomme de xanthane.

14. Une composition selon l'une des revendications 1 à 8, dans laquelle le système liquide se présente sous une forme sélectionnée parmi le groupe composé de shampooings, de gels douche et de bain.

## Fig.1.

### TYPICAL LOG VISCOSITY VS LOG CONCENTRATION PLOT FOR AN AQUEOUS POLYMER SOLUTION

## Fig.2.

### TYPICAL SIGMOID CURVE FOR A GUM-TYPE POLYMER JAGUAR HP60 AT pH 9-10

14

## Fig.3.

TYPICAL SIGMOID CURVE FOR A CROSS-LINKED POLYACRYLATE:
PPE1042 AT pH 9-10

## Fig.4.

LOG VISCOSITY VS LOG CONCENTRATION
FOR SHELLFLO XA AT pH 9-10

15

*Fig.5.*

LOG VISCOSITY VS LOG CONCENTRATION
FOR CARBOPOL 940 AT pH 9-10

*Fig.6.*

LOG VISCOSITY VS LOG CONCENTRATION
FOR CARBOPOL 910 AT pH 9-10

## *Fig.7.*

VARIATION IN SYNERGY WITH CARBOPOL 910 LEVEL
FOR A 0.1% SHELLFLO XA SOLUTION IN THE PRESENCE/ABSENCE OF NaCl

LEGEND
- ■ 3% NaCl
- □ NO SALT

## *Fig.8.*

LOG VISCOSITY VS LOG CONCENTRATION
FOR PPE1042 AT pH 9-10